# DEMANDE DE BREVET EUROPEEN

(11) **EP 1 870 057 A1**
(43) Date de publication de la demande: **26.12.2007**
(21) Numéro de dépôt: 07110432.7
(22) Date de dépôt: 18.06.2007
(51) Int. Cl.: A61F 2/06

(54) **Endoprothèse du type "stent"**

(30) Priorité: 21.06.2006 FR 0605562
(71) Demandeur: Morel d'Arleux, Eric, 93170 Bagnolet (FR)
(72) Inventeur: Morel d'Arleux, Eric, 93170 Bagnolet (FR)
(74) Mandataire: Colas, Jean-Pierre

(57) **Abrégé**

Cette endoprothèse du type stent (1) comprend un treillis élastique (3) de forme sensiblement cylindrique et un lasso proximal (9p) coopérant avec l'extrémité proximale (7p) dudit treillis élastique (3) de manière qu'un effort de traction exercé sur ce lasso proximal (9p) a pour effet de restreindre le diamètre de l'extrémité proximale (7p) de cette endoprothèse.

Cette endoprothèse comprend en outre un lasso distal (9d) coopérant avec l'extrémité distale (7d) dudit treillis élastique (3) de manière qu'un effort de traction exercé sur ce lasso distal (9d) a pour effet de restreindre le diamètre de l'extrémité distale (7d) de cette endoprothèse (1).

## Description

La présente invention se rapporte à une endoprothèse du type « stent ».

Comme cela est connu en soi, une endoprothèse du type « stent » comprend un treillis élastique de forme sensiblement cylindrique éventuellement revêtu d'une membrane souple.

Un tel stent est destiné à être posé par voie endoscopique à l'intérieur de vaisseaux (veines, artères,...), tubes (oesophage, colon...) ou canaux (urètre, uretère,...) du corps humain afin d'en maintenir l'ouverture et/ou de former une paroi artificielle.

Pour permettre le déplacement d'un tel stent à l'intérieur d'un vaisseau, tube ou canal vers la direction proximale (c'est-à-dire vers l'opérateur), soit en vue de son extraction, soit en vue de l'ajustement de sa position, il est connu de prévoir un fil en boucle ou « lasso » coopérant avec la partie proximale du treillis métallique, de sorte qu'un effort de traction exercé sur ce lasso a pour effet de restreindre le diamètre de l'extrémité proximale de ce stent et de faciliter ainsi son glissement vers la direction proximale.

On constate toutefois qu'il est fréquent que les efforts de frottement exercés par les parois des vaisseaux, tubes ou canaux sur un tel stent soient importants, ce qui rend difficile le déplacement de ce stent sous l'effet d'un effort de traction sur le lasso.

La présente invention a notamment pour but de faciliter l'extraction d'un tel stent.

On atteint ce but de l'invention avec une endoprothèse du type stent comprenant un treillis élastique de forme sensiblement cylindrique et un lasso proximal coopérant avec l'extrémité proximale dudit treillis élastique de manière qu'un effort de traction exercé sur ce lasso proximal a pour effet de restreindre le diamètre de l'extrémité proximale de cette endoprothèse, cette endoprothèse comprenant en outre un lasso distal coopérant avec l'extrémité distale dudit treillis élastique de manière qu'un effort de traction exercé sur ce lasso distal a pour effet de restreindre le diamètre de l'extrémité distale de ce stent.

Grâce à ces caractéristiques, pour extraire le stent de la position dans laquelle il se trouve à l'intérieur d'un vaisseau, tube ou canal, on peut saisir le lasso distal (c'est-à-dire le plus éloigné de l'opérateur) par l'intérieur du stent, et exercer un effort de traction sur ce lasso distal vers la direction proximale, ce qui permet d'enrouler le stent à l'intérieur de lui-même, et ainsi de l'extraire de la position dans laquelle il se trouve avec le minimum de frottement sur la paroi interne du vaisseau, tube ou canal concerné.

On notera par ailleurs qu'en exerçant un effort de traction sur ce deuxième lasso vers la direction distale, on peut déplacer le stent vers la direction distale. Une telle manoeuvre n'est toutefois possible que juste après la pose, alors qu'il n'y a pas encore d'adhérence entre le stent et la paroi interne du vaisseau, tube ou canal concerné.

Suivant d'autres caractéristiques optionnelles de l'endoprothèse selon l'invention :
- ledit treillis élastique est revêtu d'une membrane souple,
- ladite endoprothèse est choisie dans le groupe comprenant les prothèses vasculaires, oesophagiennes, colo-rectales, intestinales, biliaires, urétrales.

D'autres caractéristiques et avantages de la présente invention apparaîtront à la lecture de la description qui va suivre, et à l'examen des figures annexées, dans lesquelles :
- la figure 1 est une vue d'ensemble en perspective d'un stent selon l'invention.
- la figure 2 est une vue analogue à celle de la figure 1, l'extrémité proximale du stent étant resserrée sous l'effet d'un effort de traction exercé sur le lasso proximal,
- la figure 3 est une vue en coupe de ce stent placé à l'intérieur d'un oesophage O, et
- la figure 4 est une vue analogue à la figure 3 de ce stent en cours d'enroulement à l'intérieur de lui-même en vue de son extraction de l'oesophage O.

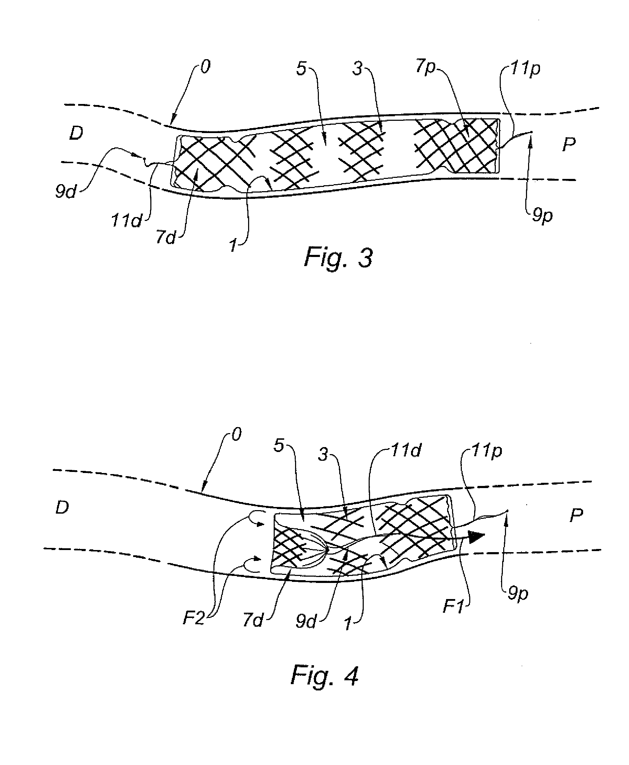

Sur les figures 3 et 4, les lettres P et D indiquent respectivement les directions proximale (c'est-à-dire vers la bouche du patient) et distale (c'est-à-dire vers l'estomac du patient) de l'oesophage.

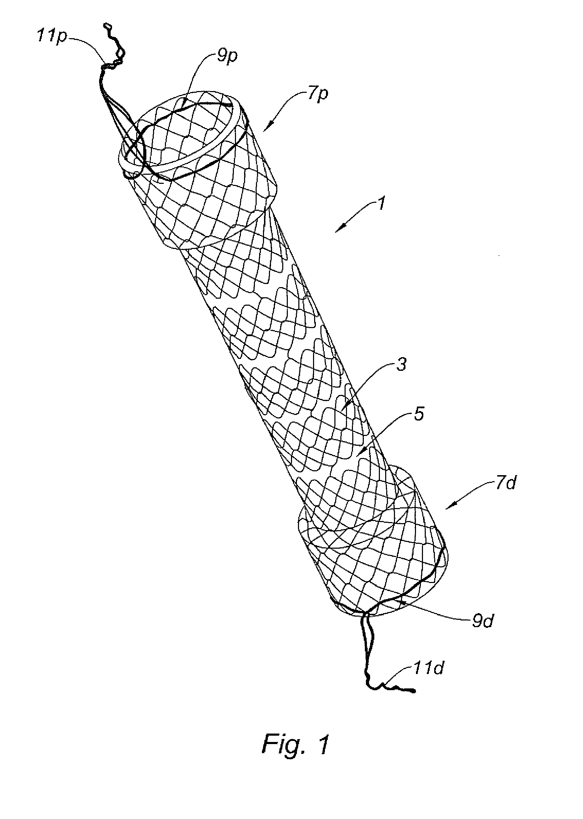

On se représente à présent à la figure 1 sur laquelle on a représenté un exemple d'un stent 1 selon l'invention.

Il s'agit en l'occurrence d'une prothèse oesophagienne, destinée à maintenir l'ouverture de l'oesophage d'un individu et/ou à former une paroi artificielle.

Comme cela est visible sur la figure 1, ce stent présente une forme générale cylindrique, dont la tenue est assurée par un treillis métallique formant ressort 3.

Ce treillis métallique est revêtu d'une membrane souple 5, formée par exemple en silicone.

A ses deux extrémités, le stent 1 comporte deux parties de plus fort diamètre, 7p et 7d destinées à être positionnées respectivement de manière proximale et distale à l'intérieur de l'oesophage d'un patient.

On notera que la présence de ces parties de plus fort diamètre est sans lien direct avec la présente invention, et que cette invention s'applique également à des stents dont les extrémités présentent toutes autres formes que celles représentées, et en particulier à des stents dont les extrémités ne sont pas évasées.

Du fil formant boucle 9p et 9d coopère avec le bord du treillis métallique 3 respectivement dans les zones de plus fort diamètre 7p et 7d.

Les fils 9p, 9d présentent chacun une partie libre 11p, 11d, permettant leur préhension par un instrument approprié.

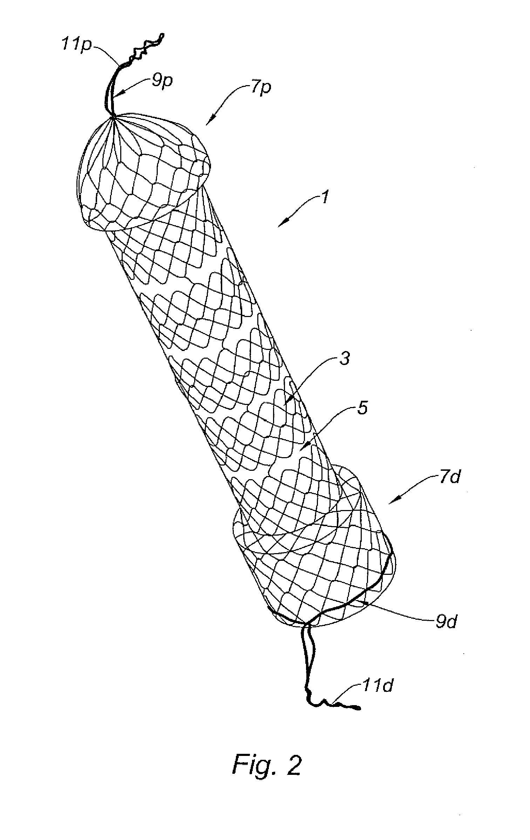

Comme cela est visible sur la figure 2, un effort de traction exercé sur la partie libre 11 p du lasso proximal 9p a pour effet de restreindre le diamètre de l'extrémité 7p du stent 1.

De même, et ceci n'étant pas représenté, un effort de traction exercé sur la partie libre 11d du lasso distal 9d a pour effet de restreindre l'extrémité distale 7d du stent 1.

Ce rétrécissement du diamètre des extrémités proximale et distale sous l'effet d'un effort de traction exercé respectivement sur les lassos proximal 9p et distal 9d permet de déplacer le stent vers les directions respectivement proximale et distale à l'intérieur de l'oesophage.

On notera toutefois qu'un tel déplacement provoque des frottements importants contre la paroi interne de l'oesophage, ce qui peut s'avérer très gênant dans certains cas, et notamment lorsque cette paroi présente des lésions ou est fragilisée.

On notera en outre que l'instrument utilisé pour exercer une traction sur les lassos, tel qu'une pince située à l'intérieur d'un endoscope, ne permet pas d'exercer une traction suffisamment forte sur les lassos pour vaincre la résistance due aux frottements du stent contre la paroi interne de l'oesophage, et en outre qu'après un séjour prolongé dans un milieu agressif, les lassos sont fragilisés et risquent de rompre sous l'effort de traction qui leur est appliqué.

On se reporte à présent aux figures 3 et 4, sur lesquelles on peut voir que le lasso distal 9d offre une possibilité particulière d'extraire le stent 1 de l'oesophage O.

Sur la figure 3, le stent 1 est représenté dans sa position normale à l'intérieur de l'oesophage O.

Comme cela est visible sur cette figure, sous l'effet de l'élasticité du treillis métallique 3, la membrane 5 de ce stent prend appui contre la paroi intérieure de l'oesophage O.

Les parties 11p, 11d des lassos proximal 9p et distal 9d demeurent libre, et peuvent ainsi être saisies par un instrument endoscopique approprié.

En particulier, et comme cela est visible sur la figure 4, la partie libre 11d du lasso distal 9d peut être saisie et ramenée à l'intérieur du stent 1 vers la direction proximale, comme cela est symbolisé par la flèche F1.

Ceci provoque un retournement du stent 1 à l'intérieur de lui-même, comme cela est symbolisé par les flèches F2.

Ce retroussement du stent permet de l'extraire de la position dans laquelle il se trouvait sans exercer le moindre effort de frottement sur la paroi interne de l'oesophage O.

Bien entendu, la présente invention n'est nullement limitée au mode de réalisation décrit et représenté, fourni à titre uniquement illustratif.

La présente invention s'étend également aux endoprothèses colo-rectales, intestinales, biliaires, urétrales, etc.

L'invention s'étend également aux endoprothèses présentant d'autres formes que celle qui a été représentée, aux endoprothèses dans lesquelles le treillis métallique n'est pas recouvert d'une membrane souple, aux endoprothèses dont le treillis métallique présente d'autres structures que celle qui a été représentée, etc.

## Revendications

1. Endoprothèse du type stent (1) comprenant un treillis élastique (3) de forme sensiblement cylindrique et un lasso proximal (9p) coopérant avec l'extrémité proximale (7p) dudit treillis élastique (3) de manière qu'un effort de traction exercé sur ce lasso proximal (9p) a pour effet de restreindre le diamètre de l'extrémité proximale (7p) de cette endoprothèse, cette endoprothèse comprenant en outre un lasso distal (9d) coopérant avec l'extrémité distale (7d) dudit treillis élastique (3) de manière qu'un effort de traction exercé sur ce lasso distal (9d) a pour effet de restreindre le diamètre de l'extrémité distale (7d) de cette endoprothèse (1),
cette endoprothèse étant **caractérisée en ce que** lesdits lassos proximal (9p) et distal (9d) comprennent chacun une partie libre (11p, 11d) permettant leur préhension par un instrument approprié.

2. Endoprothèse selon la revendication 1, dans laquelle ledit treillis élastique (3) est revêtu d'une membrane souple (5).

3. Endoprothèse selon l'une des revendications 1 ou 2, choisie dans le groupe comprenant les prothèses vasculaires, oesophagiennes, colo-rectales, intestinales, biliaires, urétrales.
